(19) Europäisches Patentamt
European Patent Office
Office européen des brevets

(11) Veröffentlichungsnummer: **0 185 971 B1**

(12) # EUROPÄISCHE PATENTSCHRIFT

(45) Veröffentlichungstag der Patentschrift: **08.11.89**

(21) Anmeldenummer: **85115250.4**

(22) Anmeldetag: **02.12.85**

(51) Int. Cl.⁴: **A 01 N 47/44**, A 61 K 7/22, A 61 K 7/16, C 11 D 3/48, A 61 K 31/35

(54) **Verwendung von Alkylglykosiden als Potenzierungsmittel in antiseptischen Mitteln sowie Desinfektions- und Reinigungsmittel mit verstärkter bakterizider Wirkung.**

(30) Priorität: **10.12.84 DE 3444958**

(43) Veröffentlichungstag der Anmeldung: **02.07.86 Patentblatt 86/27**

(45) Bekanntmachung des Hinweises auf die Patenterteilung: **08.11.89 Patentblatt 89/45**

(84) Benannte Vertragsstaaten: **AT BE CH DE FR GB IT LI LU NL SE**

(56) Entgegenhaltungen:
**DE-A-1 943 689**
**DE-A-1 965 043**
**DE-A-2 212 259**
**DE-A-2 829 037**
**DE-A-2 856 716**
**DE-B-2 036 472**

**CHEMICAL ABSTRACTS, Band 96, Nr. 21, 24. Mai 1982, Seite 79, Nr. 174391a, Columbus, Ohio, US; & JP - A - 82 09 717**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Biermann, Manfred, Dr., Markscheiderhof 25, D-4330 Mülheim/Ruhr (DE)**
Erfinder: **Lehmann, Rudolf, Dr., Schnugsheide 18, D-5653 Leichlingen (DE)**
Erfinder: **Schnegelberger, Harald, Dr., Stieglitzweg 2, D-5653 Leichlingen (DE)**
Erfinder: **Plöger, Walter, Dr., Holbeinweg 11, D-4010 Hilden (DE)**
Erfinder: **Klüppel, Hans-Jürgen, Dr., Begonienstrasse 7, D-4000 Düsseldorf (DE)**
Erfinder: **Schmid, Karl-Heinz, Dr., Stifterstrasse 10, D-4020 Mettmann (DE)**

**EP 0 185 971 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Alkylglykoside, ihre Herstellung und ihre Verwendung insbesondere als Tenside sind seit langer Zeit bekannt. Verwiesen wird beispielsweise auf die US-PSen 3 839 318, 3 707 535 und 3 547 828, die DE-OSen 1 905 523, 1 943 689, 2 036 472 und 3 001 064 sowie die EP-OS 0 077 167. Ihre Herstellung erfolgt insbesondere durch Umsetzung von Glucose oder Oligosacchariden mit Alkoholen der C-Kettenlänge von 8 bis 25 C-Atomen insbesondere als biologisch abbaubare oberflächenaktive Stoffe, die vielseitige Verwendung finden können.

Bei der Untersuchung der mikrobiologischen Eigenschaften von Alkylglykosiden zeigte sich, dass diese selbst bei hohen Anwendungskonzentrationen – zum Beispiel 10 000 ppm – alleine keine wesentlichen antimikrobiellen Wirkungen aufweisen. Auch ihre Kombination mit quartären Ammoniumverbindungen führt zu keinen bemerkenswerten Effekten. Quartäre Ammoniumverbindungen haben als solche bakterizide Wirkung. Der kombinierte Einsatz von Alkylglykosiden und quartären Ammoniumverbindungen, wie beispielsweise in der US-PS 3 547 828 beschrieben ist, führt zu keinen unerwarteten technischen Effekten.

Überraschenderweise wurde jetzt festgestellt, dass Alkyl- und/oder Alkenylglykoside in Kombination mit ausgewählten bekannten antimikrobiellen Wirkstoffen synergistische Leistungssteigerungen insbesondere bezüglich bestimmter mikrobizider Wirkungen dieser Wirkstoffe liefern. Vor allem die Wirksamkeit gegen grampositiven Bakterien der erfindungsgemäss ausgewählten Wirkstoffe wird durch ihre Kombination mit Alkyl- und/oder Alkenylglykosiden deutlich gesteigert.

Gegenstand der Erfindung ist dementsprechend die Verwendung von Alkyl- und/oder Alkenylglykosiden mit 8 bis 16 C-Atomen im Alkyl- und/oder Alkenylrest in Abmischung mit bakterizid wirksamen Biguanidverbindungen zur Potenzierung der mikrobiziden Wirksamkeit der Biguanidverbindungen, insbesondere gegen grampositive Bakterien, in antiseptischen Mitteln. Die Erfindung betrifft insbesondere Desinfektions- und Reinigungsmittel mit bakterizider Wirkung, die durch diese Kombination von Alkyl- und/oder Alkenylglykosiden mit den genannten mikrobiziden Wirkstoffen gekennzeichnet sind. Wichtig sind hier insbesondere entsprechende Mittel zur Körperpflege wobei Mittel zur Pflege des Mund- und Zahnbereichs, insbesondere Zahnpasten, Zahnpulvern und Mundwassern besondere Bedeutung bekommen kann.

Mikrobizide Wirkstoffe für die Abmischung mit Alkyl- und/oder Alkenylglykosiden sind im Rahmen der Erfindung antiseptische Biguanidverbindungen, wobei insbesondere Chlorhexidinsalze bedeutungsvoll sein können. Chlorhexidin ist bekanntlich der internationale Freiname für das antiseptisch wirkende 1,1'-Hexamethylenbis-[5-(4-Chlorphenyl)-biguanid], das in Form seiner Salze – zum Beispiel als Acetat, Hydrochlorid oder als Glukonat – weitverbreitete Anwendung im kosmetischen und pharmazeutischen Bereich sowie für Reinigungszwecke gefunden hat. Andere bekannte Desinfektionsmittel auf Biguanidbasis sind beispielsweise die Salze von Polyhexamethylenbiguanidverbindungen der allgemeinen Formel

SEITE 3

$$HX\cdot NH_2(CH_2)_3-\left[(CH_2)_3-NH\underset{\underset{NH}{\|}}{C}-NH-\underset{\underset{NH\cdot HX}{\|}}{C}-NH-(CH_2)_3\right]_n-(CH_2)_3-NH-\underset{\underset{NH}{\|}}{C}-NH-CN \qquad (I)$$

Hierbei ist HX die salzbildende Säurekomponente, zum Beispiel HC1, während n eine Zahl von wenigstens 2 und beispielsweise eine Zahl von etwa 4,5 bis 6,5 ist.

Antimikrobiell wirksame Biguanidverbindungen sind in der einschlägigen Patentanliteratur zahlreich erwähnt worden. Verwiesen sei beispielsweise auf die EP-OS 0 024 031 und die dort auf Seite 36 bis 43 erwähnten US-PSen 2 684 924, 2 990 425, 3 468 898, 4 022 834 und 4 053 636. Verwiesen wird weiterhin auf die DE-OSen 22 12 259 und 26 27 548, wobei im einzelnen die folgenden weiteren Beispiel genannt seien: $N^1$-(4-Chlorbenzyl)-$N^5$-(2,4-dichlorbenzyl)-biguanid; p-Chlorphenyl-biguanid; 4-Chlorbenzylhydryl-biguanid; N-3-Lauroxypropyl-$N^5$-p-chlorbenzyl-biguanid; $N^1$-p-Chlorphenyl- $N^5$-laurylbiguanid sowie deren nichttoxische Additionssalze insbesondere Glukonate oder Acetate.

Die überraschende Wirkungssteigerung der erfindungsgemässen Lehre wird beispielsweise aus folgenden Zahlen ersichtlich; 250 ppm Chlorhexidinglukonat in wässriger Lösung (17° dH, 20 °C) reichen für eine Abtötung von Staphylococcus aureus innerhalb einer Stunde noch nicht aus. Durch Zusatz von 0,1% Alkylglykosid zur wässrigen Lösung wird eine Abtötung von Staphylococcus aureus schon mit 50 ppm Chlorhexidinglukonat innerhalb von 15 Minuten erreicht. Ähnlich sieht das Wirkungsverhältnis gegenüber Streptococcus faecium aus.

Für den Einsatz der genannten antimikrobiellen Wirkstoffe auf wichtigen Anwendungsgebieten erschliesst sich damit die Möglichkeit, mit deutlich verringerten Wirkstoffgehalten arbeiten zu können, und gleichwohl befriedigende mikrobizide Wirkungen, insbesondere gegenüber grampositiven Bakterien zu erzielen. Im bereits erwähnten Gebiet der Mund- und Zahnpflegemittel wird bekanntlich insbesondere der Wirkung von grampositiven Bakterien Beachtung geschenkt. Die beispielsweise in Zahnpasten zugelassenenen bakteriostatischen Mittel können in den erlaubten Konzentrationen eine echte Antiplaquewirkung bis heute nicht erzielen. Bekannt ist dabei weiterhin, dass bei der Bildung des Zahnbelags und der dadurch mit ausgelösten Kariesentwicklung grampositive Bakterien eine besondere Rolle spielen. Die erfindungsgemässe Möglichkeit der Wirkungsverstärkung gerade gegenüber den grampositiven Bakterien erschliesst hier den Zugang zu weitaus wirkungsvolleren Mitteln, als sie bisher zur Verfügung standen, ohne dass es einer Erhöhung der Wirkstoffkonzentration bedarf.

Häufig gilt für die Erfindung die allgemeine Lehre, dass die Alkyl- und/oder Alkenylglykoside wenigstens in etwa gleichen Gewichtsmengen wie die bakterizid wirksamen Komponenten eingesetzt werden. Durch einfache Vorversuche lassen sich jeweils besondere geeignete Mischungsverhältnisse von mikrobizidem Wirkstoff und Alkyl- und/oder Alkenylglykosid feststellen. Es kann aber auch zweckmässig sein, mit vergleichsweise geringen Mengen der Alkyl- und/oder Alkenylglykoside zu arbeiten.

Die Alkyl- und/oder Alkenylglykoside können allerdings auch in gewichtsmässig beträchtlichem Überschuss über die bakterizid wirkenden Komponenten eingesetzt werden. Das kann für die nachfolgend geschilderte besonders wichtige Anwendungsform der hier offenbarten synergistischen Wirkungssteigerung im Rahmen von Desinfektions- und Reinigungsmittel insbesondere des Mund- und Zahnbereichs von besonderer Bedeutung sein.

Die erfindungsgemäss als Potenzierungsmittel eingesetzten Alkyl- und/oder Alkenylglykoside sind beispielsweise aus den eingangs genannten Druckschriften bekannt. Sie leiten sich insbesondere von Fettalkoholen und Zuckern ab. Bevorzugt sind Alkyl- und/oder Alkenylglykoside mit einem $C_6$-, $C_8$-, $C_{10}$-, $C_{12}$-, $C_{14}$-, $C_{16}$- und/oder $C_{18}$-Alkyl- und/oder Alkenylrest, der geradkettig oder verzweigt sein kann, wobei ein Alkenylrest einfach oder mehrfach olefinisch ungesättigt sein und beispielsweise bis zu 3 Doppelbindungen aufweisen kann, oder Mischungen derartiger Produkte. Besonders wichtig sind Alkyl- und/oder Alkenylglykoside, deren Alkyl- oder Alkenylrest insbesondere 8–16 C-Atome und vor allem 10–14 C-Atome aufweist. Bezüglich des Saccharidrestes der Alkyl- und/oder Alkenylglykoside gilt, dass sowohl Alkyl- und/oder Alkenyl-monoglykoside, bei denen ein cyclischer Zuckerrest an den Fettalkohol gebunden ist, geeignet sind, aber auch Oligomere mit vorzugsweise bis zu 8, insbesondere bis zu 3 glykosidisch gebundenen Glukose- oder Maltoseresten. Dabei ist die Anzahl der Zuckerreste ein statistischer Mittelwert, dem die bei diesem Produkten übliche Verteilung zugrunde liegt. Alkyl- und/oder Alkenylglykoside mit $C_{10}$- bis $C_{14}$-Fettalkoholen im Alkyl- und/oder Alkenylrest und mit bis zu 2 Glykosidresten, vorzugsweise mit bis zu 1,5 Glykosidresten, können besonders geeignet sein.

Die erfindungsgemässe bakterizide Wirkungssteigerung findet eine ihrer wichtigsten Anwendungen im Rahmen von Körperpflegemitteln insbesondere bei Desinfektions- und Reinigungsmitteln des Mund- und Zahnbereichs. Zum allgemeinen Aufbau solcher Mittel sei beispielsweise verwiesen auf die bereits genannten DE-OSen 22 12 259 und 26 27 548. Solche Mittel können – bezogen auf das Gewicht – etwa 0,01% bis etwa 5%, vorzugsweise nicht über 2,5 Gewichtsprozent der mikrobiziden Biguanid-Wirkstoffe enthalten. Besonders geeignete Zusatzmengen der Biguanidverbindungen liegen im Bereich von etwa 0,03 bis etwa 1,2% und insbesondere im Bereich von etwa 0,05 bis etwa 0,8%, wobei sich alle diese Zahlenwerte jeweils auf das Gewicht der gesamten Zubereitung beziehen. In Abmischung damit liegen die Alkyl- und/oder Alkenylglykoside vor, die bevorzugt in wenigstens etwa gleichen Mengen ggf. aber auch in beträchtlichem Überschuss zugegeben sind. Da Alkyl- und/oder Alkenylglykoside als solche bekannte Tenside sind, können sie beispielsweise im Rahmen von Zahnpasten oder Zahnpulvern dort gleichzeitig die Tensidfunktion übernehmen und dann in entsprechenden Mengenverhältnissen zum Einsatz kommen.

In einer wichtigen Ausführungsform wird die Menge der Alkyl- und/oder Alkenylglykoside auf Konzentrationen nicht über 2 000 ppm und insbesondere auf Werte nicht über 1 000 ppm begrenzt. Schon sehr geringe Mengen von z.B. etwa 10 ppm und vorzugsweise von wenigstens etwa 50 ppm geben mit den Biguanidverbindungen deutliche Wirkungsverstärkungen, so dass in entsprechenden Wirkstofflösungen Alkyl- und/oder Alkenylglykosidkonzentrationen im Bereich von etwa 50–500 ppm besonders bevorzugt sein können.

Der pH-Wert solcher Körperpflegemittel wird vorzugsweise im Bereich von etwa 4,5 bis etwa 9,5 gehalten, üblicherweise liegt er im Bereich von etwa 6 bis 8. Die erfindungsgemässe Wirkstoffkombination wird zusammen mit geeigneten Trägerkomponenten eingesetzt, die ihrerseits an sich bekannten Funktionen solcher Reinigungs- und Pflegemittel aufweisen können. Vorzugsweise ist die Trägerkomponente des Mund- und/oder Zahnpflegemittels eine übliche Zahnpaste, Mundspülzubereitung, Kaugummimasse oder dergleichen.

Zahnpflege- bzw. Zahnputzmittel enthalten ein schleifend bzw. scheuernd wirkendes Poliermittel und normalerweise Schaumbildner, Geschmacksstoff und Süssmittel. Zahnpasten enthalten ausserdem meist Feuchthaltemittel, Bindemittel und Wasser. Bekannte geeignete Poliermittel sind beispielsweise Calziumkarbonat, Dicalziumorthophosphat-Dihydrat, Calziumpyrophosphat, Calziumpolymetaphosphat und unlösliches Natriumpolymetaphosphat, Aluminiumtrihydroxid, α-Aluminiumoxid und Kieselsäuren, insbesondere Gelkieselsäuren und Fällungskieselsäuren. Die Verwendung von Schleif- bzw. Scheuermitteln, die mit den Biguanidverbindungen kompatibel sind, kann besonders bevorzugt sein. Auch hierzu wird auf die Angaben des Standes der Technik verwiesen, wie sie beispielsweise in der DE-OS 26 27 548, dort Seite 6 letzter Absatz, angegeben sind.

Der Gesamtanteil an Schleif- bzw. Scheuermittel kann in den als Zahnputzmittel geeigneten erfindungsgemässen Zubereitungen etwa 0,5 bis 95% des Gewichts des Zahnputzmittels betragen. Üblicherweise liegt ihr Anteil bei Zahnpasten im Bereich von 6 bis 60 Gewichtsprozent, bei Zahnpulvern im Bereich von etwa 20 bis etwa 95%.

Zahnpflege- bzw. Zahnputzmittel enthalten üblicherweise Tenside als Schaumbildner. Es können übliche nicht-seifenartige, nichtionische, kationische, zwitterionische und amphotere organische synthetische Tenside verwendet werden. Besonders geeignet sind jedoch wie bereits erwähnt die erfindungsgemäss als Wirkungsverstärker eingesetzten Alkyl- und/oder Alkenylglykoside. Geeignete nichtionische Detergentien sind die Kondensationsprodukte von Alkylenoxiden mit organischen hydrophoben Verbindungen, die beispielsweise einen aliphatischen oder alkylaromatischen Rest enthalten. Die Schaumbildner werden in Zahnputz- bzw. Zahnpflegemitteln üblicherweise in Mengen von etwa 0,5 bis 5 Gewichtsprozent eingesetzt.

Geeignete Geschmacksstoffe sind beispielsweise Methylsalizilat, Pfefferminzöl, Sassafraöl und Anisöl. Geschmacksstoffe werden meist in Mengen von etwa 0,01 bis 2,0 Gewichtsprozent verwendet. Die üblichen Süssmittel bzw. Süssstoffe können in Mengen von etwa 0,05 bis etwa 2 Gewichtsprozent verwendet werden.

Zur möglichen Mitverwendung von Verdikkungsmitteln wie Hydroxyethylzellulose und wasserlöslichen Salzen von Zelluloseethern, natürlichen Gummen bzw. Pflanzenschleimen oder kolloidalen anorganischen Komponenten, wie feinzerteiltes Siliziumoxid oder kolloidales Magnesiumaluminiumsilikat, wird auf den einschlägigen bereits zitierten Stand der Technik verwiesen. Verdickungsmittel liegen häufig in Mengen von etwa 0,1 bis 5,0 Gewichtsprozent einer Zahnpastenzubereitung vor. Geeignete Feuchthaltemittel sind beispielsweise Glycerin, Sorbit und andere mehrwertige Alkohole oder Mischungen hiervon. Sie können in Mengen von etwa 1 bis etwa 50 Gewichtsprozent der Zahnpastenzubereitung vorliegen und sind zumeist in Abmischung mit Wasser zugegeben.

Mundspülmittel enthalten meist eine Wasser/Ethylalkohol-Lösung und gewünschtenfalls weitere Komponenten wie Geschmacksstoffe, Süssmittel und Feuchthaltemittel der erwähnten Art. Im erfindungsgemässen Sinn enthalten sie die geschilderte Kombination von antimikrobiell wirksamen Biguanidverbindungen und Alkyl- und/oder Alkenylglykosiden.

Allgemein ist dabei das folgende zu beachten: Durch Mitverwendung sonstiger Komponenten, insbesondere von Tensiden und/oder Emulgatoren, kann eine Verschlechterung des erfindungsgemässen Effekts der Wirkungssteigerung bis hin zur Unwirksamkeit ausgelöst werden. Durch Vorversuche ist dabei aber stets der geeignete Mischungsbereich der im Einzelfall vorliegenden Komponenten zu ermitteln. Wichtig ist dabei, dass Alkyl- und/oder Alkenylglykoside selber Tensidcharakter besitzen und damit die Einschränkung der Menge anderer Tenside bzw. Emulgatoren leicht möglich wird.

Beispiele

A. Mikrobizide Wirksamkeit

Die mikrobizide Wirksamkeit der erfindungsgemässen Kombinationen aus bakterizid wirksamen Biguanidverbindungen und Alkylglylosiden wurde gegenüber folgenden Testkeimsuspensionen bestimmt:

| | |
|---|---|
| A) Staphylococcus aureus | $2 \times 10^9$ Keime/ml |
| B) Streptococcus faecium | $2 \times 10^9$ Keime/ml |
| C) Streptococcus mutans | $1 \times 10^9$ Keime/ml |
| D) Escherichia coli | $2 \times 10^9$ Keime/ml |
| E) Candida albicans | $2 \times 10^9$ Keime/ml |

Die Abtötungszeiten der zu untersuchenden Produkte wurden mit Hilfe des Suspensionstestes ermittelt. Unter Verwendung von Wasser einer Härte von 17° dH wurden Testlösungen hergestellt, die neben 1000 bzw. 100 ppm Alkylglykosid 500, 250, 100, 50 und 25 ppm Chlorhexidinglukonat (1,1'-Hexamethylenbis-[4-chlorphenyl)-biguanid]- glukonat) enthielten. Daneben wurden Vergleichslösungen hergestellt, die einerseits nur Chlorhexidinglukonat in den angegebenen Konzentrationen, und andererseits nur die jeweiligen Alkylglykoside in einer Konzentration von 10 000 ppm enthielten.

Bei Raumtemperatur wurden jeweils 0,1 ml Testkeimsuspension in Reagenzgläser pipettiert und mit jeweils 10 ml der oben beschriebenen Test- oder Vergleichslösungen vermischt. Nach unterschiedlichen Einwirkungszeiten bis zu 60 Minuten wurden den Reagenzgläsern mit Hilfe einer Impföse ca. 0,05 ml Material entnommen und auf einem Nähragar, der als Enthemmer 3% Tween 80 und 0,3% Lecithin enthielt, ausgestrichen. Das Nährmedium bestand für die Keime A bis D aus 2,5 gewichtsprozentiger Standard-I-Bouillon (Merck), und für den Keim E aus Würzebouillon pH 5 (Merck), die zur Verfestigung jeweils 1,2 Gewichtsprozent Agar enthielten. Die Proben wurden bei 37° bzw. 30 °C bebrütet. Nach frühestens 3 Tagen wurden die Kulturen makroskopisch auf Wachstum beurteilt und auf diesem Weg die Abtötungszeit oder der Restkeimgehalt ermittelt.

In den nachfolgenden Tabellen bedeuten «+» weniger als 50, «++» weniger als 200 und «+++» mehr als 200 Restkeime nach 60 Minuten Einwirkungszeit.

Beispiel 1

In einer ersten Versuchsreihe wurden die folgenden Alkylglykoside 1 bis 6 jeweils in Konzentrationen von 1000 ppm neben den in der Tabelle I angegebenen Mengen Chlorhexidinglukonat eingesetzt:

1) ein $C_8/C_{10}$-Alkyl-oligoglulosid (Oligomerisierungsgrad 1,8), dessen Alkylreste sich von einem n-Octanol/n-Decanolgemisch im Gewichtsverhältnis 60:40 ableiten

2) ein $C_8/C_{10}$-Alkyl-oligoglukosid (Oligomerisierungsgrad 1,8), dessen Alkylreste sich von einem n-Octanol/n-Decanolgemisch im Gewichtsverhältnis 40:60 ableiten

3) ein $C_8/C_{10}$-Alkyl-oligoglukosid (Oligomerisierungsgrad 1,3), dessen Alkylreste sich von einem n-Octanol/n-Decanolgemisch im Gewichtsverhältnis 50:50 ableiten

4) ein $C_{12}/C_{14}$-Alkyl-monoglukosid, dessen Alkylreste sich von e inem n-Dodecanol/n-Tetradecanolgemisch im Gewichtsverhältnis 70:30 ableiten

5) ein $C_{12}/C_{14}$-Alkyl-oligoglukosid (Oligomerisierungsgrad 1,5), dessen Alkylreste sich von einem n-Dodecanol/n-Tetradecanolgemisch im Gewichtsverhältnis 70:30 ableiten

6) ein $C_{12}/C_{14}$-Alkyl-oligoglukosid (Oligomerisierungsgrad 1,4), dessen Alkylreste sich von einem n-Dodecanol/n-Tetradecanolgemisch im Verhältnis 70:30 ableiten

Die Lösungen wurden gegenüber Suspensionen der Keime A, B und C getestet. Die gefundenen Ergebnisse sind in der nachfolgenden Tabelle I wiedergegeben. In der mit «0» bezeichneten ersten Spalte ist die Wirkung der Chlorhexidinglukonatlösungen ohne Zusatz von Alkylglukosid verzeichnet.

Tabelle I

| Chlorhexidin-Glukonat (ppm) | Staphylococcus aureus 0 | 1 | 2 | 3 | 4 | 5 | 6 |
|---|---|---|---|---|---|---|---|
| 500 | 60 | – | – | – | – | – | – |
| 250 | + | – | – | – | ≤15 | ≤15 | ≤15 |
| 100 | +++ | 15 | ≤15 | ≤15 | 60 | ≤15 | ≤15 |
| 50 | – | 15 | ≤15 | ≤15 | + | 60 | ≤15 |
| 25 | – | 15 | 15 | ≤15 | – | – | – |
| 0* | | + | + | + | +++ | +++ | +++ |
| | Streptococcus faecium | | | | | | |
| 500 | ++ | – | – | – | – | – | – |
| 250 | +++ | – | – | – | ≤15 | ≤15 | ≤15 |
| 100 | +++ | ≤5 | ≤5 | ≤5 | 60 | ≤15 | ≤15 |
| 50 | – | ≤5 | ≤5 | ≤5 | +++ | 60 | ≤15 |
| 25 | – | ≤5 | 15 | ≤5 | – | – | – |
| 0* | | +++ | +++ | +++ | +++ | +++ | +++ |
| | Streptococcus mutans | | | | | | |
| 500 | ++ | – | – | – | – | – | – |
| 250 | ++ | – | – | – | – | – | ≤15 |
| 100 | +++ | 60 | 60 | 60 | – | – | 60 |
| 50 | – | 60 | 60 | 60 | – | – | 60 |
| 25 | – | 60 | 60 | 60 | – | – | – |
| 0* | | ++ | ++ | ++ | – | – | ++ |

*) Eigenwirkung der Alkylglukoside bei 10 000 ppm

Beispiel 2

In einer zweiten Versuchsreihe wurden die folgenden Alkylglukoside 7 bis 10 jeweils in Konzentrationen von 100 ppm neben den in der Tabelle II angegebenen Mengen Chlorhexidinglukonat eingesetzt:

7) ein $C_{12}/C_{14}$-Alkyl-oligoglukosid (Oligomerisierungsgrad 1,4), dessen Alkylreste sich von einem n-Dodecanol/n-Tetradecanolgemisch im Gewichtsverhältnis 70:30 ableiten

8) ein $C_{12}/C_{14}$-Alkyl-oligoglukosid (Oligomerisierungsgrad 1,7), dessen Alkylreste sich von einem n-Dodecanol/n-Tetradecanolgemisch im Gewichtsverhältnis 70:30 ableiten

9) ein Dodecyl-monoglukosid

10) ein Undecenyl-monoglukosid

Die Lösungen wurden gegenüber Suspensionen der Keime A, D und E getestet. Die gefundenen Ergebnisse sind in der nachfolgenden Tabelle II wiedergegeben. Auch hier ist die Wirkung der Chlorhexidinlösungen ohne Zusatz von Alkylglukosid in der mit «0» bezeichneten zweiten Spalte der Tabelle verzeichnet.

Tabelle II

| Chlorhexidin-Glukonat (ppm) | Staphylococcus aureus | | | | |
|---|---|---|---|---|---|
| | 0 | 7 | 8 | 9 | 10 |
| 500 | 60 | – | – | – | – |
| 250 | ++ | – | – | – | – |
| 100 | +++ | 15 | 15 | 15 | 60 |
| 50 | – | 15 | 15 | 15 | 60 |
| 25 | – | 60 | 15 | 15 | ++ |
| 0* | | +++ | +++ | +++ | ++ |
| | Escherichia coli | | | | |
| 500 | 15 | – | – | – | – |
| 250 | 15 | – | – | – | – |
| 100 | 60 | ⩽5 | ⩽5 | ⩽5 | ⩽5 |
| 50 | ++ | 60 | 60 | 60 | 15 |
| 25 | – | +++ | ++ | + | 60 |
| 0* | | +++ | +++ | +++ | +++ |
| | Candida albicans | | | | |
| 500 | ⩽5 | – | – | – | – |
| 250 | 15 | – | – | – | – |
| 100 | 60 | ⩽5 | 15 | 15 | 15 |
| 50 | + | 60 | 60 | 15 | 60 |
| 25 | ++ | 60 | + | 60 | 60 |
| 0* | | 60 | 60 | +++ | +++ |

*) Eigenwirkung der Alkylglukoside bei 10 000 ppm

B. Wirksamkeit bei der Flächendesinfektion

Beispiel 3

Die Wirksamkeit der erfindungsgemässen Kombination aus bakterizid wirksamen Biguanidverbindungen und Alkylglukosiden im Flächendesinfektionstest wurde nach den Richtlinien für die Prüfung chemischer Desinfektionsverfahren der Deutschen Gesellschaft für Hygiene und Mikrobiologie beschrieben in Hygiene + Medizin 9 (1984), S. 42–43, geprüft.

Die verwendete Testkeimsuspension enthielt Staphylococcus aureus in einer Konzentration von $2 \times 10^9$ Keimen/ml.

Als erfindungsgemässe Mittel wurden die Lösungen A und B eingesetzt, die beide 500 ppm Chlorhexidinglukonat und 1000 ppm Isotridecyloligoglukosid (Oligomerisierungsgrad 1,5) enthielten. Lösung B enthielt zusätzlich 1000 ppm Analgerungsprodukt von 5 Mol Ethylenoxid und 4 Mol Propylenoxid an einen $C_{12}$–$C_{14}$-Fettalkohol (Zusammensetzung in Gewichtsprozent: 0–2 $C_{10}$; 70–75 $C_{12}$; 25–30 $C_{14}$; 0–2 $C_{16}$). Die Lösungen A und B wurden gegenüber Wasser einer Härte von 17° dH (Wasser standardisierter Härte = WSK) und gegenüber der Vergleichslösung C, die nur 500 ppm Chlorhexidinglukonat enthielt, geprüft.

Als Testflächen wurden 50×50 mm grosse Bodenbelagsstücke aus Weich-PVC nach DIN 16 951 (Ausgabe April 1977) eingesetzt. Vor der Kontamination wurden die Testflächen zuerst mit Wasser, dann mit Ethanol (70 Volumenprozent) abgerieben und getrocknet.

Auf jede Testfläche wurden 0,1 ml Keimsuspension mit einer Pipette aufgebracht und mit einem Glasspatel auf dem mittleren Areal von 30×30 mm Kantenlänge gleichmässig verteilt. Nach dem Antrocknen der Keimsuspension im Verlauf von 90 Minuten wurden auf den Testflächen jeweils 0,2 ml der oben angegebenen Lösungen mit einem Glasspatel verteilt.

Nach einer Einwirkungszeit von 15 bzw. 60 Minuten wurden die PVC-Stücke in 200 ml-Behälter mit Verschluss gebracht, die 100 ml Casein-Soja-Pepton-Lösung mit 3 Gewichtsprozent Tween 80 und 0,3 Gewichtsprozent Lecithin und Glaskugeln enthielten. Zum Abschwemmen der Keime wurden die Glasbehälter 2 Minuten lang auf einer Schüttelmaschine geschüttelt. Danach wurden aus den erhaltenen Flüssigkeiten jeweils zwei Verdünnungen ($10^{-2}$ und $10^{-4}$) hergestellt. Von den Flüssigkeiten und den Verdünnungen wurden jeweils 0,1 ml auf einer Platte aus Casein-Soja-Pepton-Agar, der 3 Gewichtsprozent Tween 80 und 0,3 Gewichtsprozent Lecithin enthielt, mit einem Spatel verteilt.

Die Agarplatten wurden bei 37 °C bebrütet. Nach frühestens 2 Tagen wurde die Anzahl der koloniebildenden Einheiten (KBE) bestimmt.

In der nachfolgenden Tabelle III ist für die einzelnen Lösungen jeweils die Keimzahlreduktion $KR_t$ für eine bestimmte Einwirkungszeit (hier 15 und 60 Minuten) im Vergleich zu WSH wiedergegeben. Die Keimreduktion wird berechnet nach der Formel

$$KR_t = \log KBE\,(WSH) - \log KBE\,(D)$$

in der KBE (WSH) die Anzahl koloniebildender Einheiten nach Einwirkung von WSH und KBE. (D) die Anzahl kolonienbildender Einheiten nach Einwirkung des Desinfektionsmittels bedeuten.

Tabelle III

| Lösung | $KR_{15}$ | $KR_{60}$ |
|---|---|---|
| A) 500 ppm Chlorhexidinglukonat<br>1000 ppm Isotridecyloligoglukosid | 3,0 | 3,7 |
| B) 500 ppm Chlorhexidinglukonat<br>1000 ppm Isotridecyloligoglukosid<br>1000 ppm Fettalkohol + 5 EO +4 Po | 2,4 | 3,0 |
| C) 500 ppm Chlorhexidinglukonat | 2,1 | 2,4 |

## Patentansprüche

1. Verwendung von Alkyl- und/oder Alkenylglykosiden mit 8 bis 16 C-Atomen im Alkyl- und/oder Alkenylrest in Abmischung mit bakterizid wirksamen Biguanidverbindungen zur Potenzierung der mikrobiziden Wirksamkeit der Biguanidverbindungen, insbesondere gegen grampositive Bakterien, in antiseptischen Mitteln.

2. Ausführungsform nach Anspruch 1, dadurch gekennzeichnet, dass die Alkyl- und/oder Alkenylglycoside in Abmischung mit Chlorhexidinsalzen verwendet werden.

3. Ausführungsform nach Ansprüchen 1 und 2, dadurch gekennzeichnet, dass die Alkyl- und/oder Alkenylglykoside wenigstens in etwa gleichen Gewichtsmengen wie die bakterizid wirksamen Komponenten eingesetzt werden, wobei Mengen unter 2000 ppm und insbesondere unter 1000 ppm bevorzugt sein können.

4. Ausführungsform nach Ansprüchen 1 bis 3, dadurch gekennzeichnet, dass Alkyl- und/oder Alkenylglykoside mit 10 bis 14 C-Atomen im Alkylrest verwendet werden.

5. Ausführungsform nach Ansprüchen 1 bis 4, dadurch gekennzeichnet, dass Alkyl- und/oder Alkenyl-mono- und/oder Alkyl- und/oder Alkenyl-polyglykoside mit bis zu 8 Glykosidresten, insbesondere mit bis zu 3 Glykosidresten verwendet werden.

6. Desinfektions- und Reinigungsmittel mit insbesondere grampositiver bakterizider Wirkung, insbesondere entsprechende Mittel zur Körperpflege wie Zahnpasten, Zahnpulver und Mundwasser, gekennzeichnet durch einen Gehalt an Wirkstoffgemischen aus Alkyl- und/oder Alkenylglykosiden und bakteriziden Wirkstoffen gemäss Ansprüchen 1 bis 5.

## Revendications

1. Utilisation dans des antiseptiques, d'alkylet/ou d'alcénylglucosides dont le résidu alkyle et/ou alcényle comporte 8 à 16 atomes de carbone, en mélange avec des composés biguanides à pouvoir bactéricide, en vue de potentialiser l'activité microbicide desdits composés biguanides, en particulier vis-à-vis des bactéries Gram positif.

2. Version selon la revendication 1, caractérisée en ce que les alkyl- et/ou alcénylglucosides sont utilisé en mélange avec des sels de chlorhexidine.

3. Version selon les revendications 1 et 2, caractérisée en ce que les alkyl- et/ou les alcénylglucosides sont utilisés dans des quantités en poids au moins à peu près équivalentes à celles des composants à action bactéricide, des quantités inférieures à 2000 ppm et en particulier, inférieures à 1000 ppm pouvant être préférées.

4. Version selon les revendications 1 à 3, caractérisée en ce que des alkyl- et/ou alcénylglucosides dont le résidu alkyle comporte 10 à 14 atomes de carbone sont utilisés.

5. Version selon les revendications 1 à 4, caractérisée en ce que des alkyl- et/ou alcénylmonoglucosides et/ou des alkyl- et/ou alcénylpolyglucosides comportant jusqu'à 8 résidus glucoside, en particulier jusqu'à 3 résidus glucoside, sont utilisés.

6. Désinfectants et nettoyants possédant en particulier un pouvoir bactéricide vis-à-vis des germes Gram positif, en particulier produits correspondants pour les soins corporels, tels que pâtes, poudres et eaux dentifrices, se caractérisant par une concentration en mélanges de substances actives constituées d'alkyl- et/ou d'alcénylglucosides et d'agents bactéricides, selon les revendications 1 à 5.

## Claims

1. The use of alkyl and/or alkenyl glycosides containing 8 to 16 C atomes in the alkyl and/or alkenyl radical in admixture with bactericidal biguanide compounds for potentiating the microbicidal activity of the biguanide compounds, particularly against gram-positive bacteria, in antiseptic preparations.

2. The use claimed in claim 1, characterized in that the alkyl and/or alkenyl glucosides are used in admixture with chlorhexidine salts.

3. The use claimed in claims 1 and 2, characterized in that the alkyl and/or alkenyl glycosides are used in at least substantially the same quantities by weight as the bactericidal components, quantities below 2000 ppm being preferred and quantities below 1000 ppm particularly preferred.

4. The use claimed in claims 1 to 3, characterized in that alkyl and/or alkenyl glycosides containing 10 to 14 C atoms in the alkyl radical are used.

5. The use claimed in claims 1 to 4, characterized in that alkyl and/or alkenyl mono- and/or alkyl and/or alkenyl polyglycosides containing up to 8 and more especially up to 3 glycoside groups are used.

6. Disinfecting and cleaning preparations effective in particular against gram-positive bacteria, more especially corresponding personal hygiene preparations, such as toothpaste, tooth powders and mouthwashes, characterized in that they contain mixtures of alkyl and/or alkenyl glycosides glycosides and bactericidal compounds of the type defined in claims 1 to 5.